# EUROPÄISCHE PATENTSCHRIFT

(11) **EP 0 531 802 B1**
(45) Veröffentlichungstag und Bekanntmachung des Hinweises auf die Patenterteilung: **20.12.1995**
(21) Anmeldenummer: 92114572.8
(22) Anmeldetag: 27.08.1992
(51) Int. Cl.: A61K 6/083, C09J 133/14

(54) **Adhäsivkomponente zur Restaurierung der Zahnhartsubstanz**
Adhesive for the restauration of hard dental material
Adhésif pour la restauration de la matière dure dentaire

(30) Priorität: 09.09.1991 DE 4129877
(43) Veröffentlichungstag der Anmeldung: 17.03.1993
(73) Patentinhaber: Heraeus Kulzer GmbH & Co.KG, 63450 Hanau (DE)
(72) Erfinder: Müller, Michael, Dr., W-5060 Bergisch Gladbach 2 (DE); Podszun, Wolfgang, Dr., W-5000 Köln 80 (DE); Finger, Werner, Prof. Dr., W-4040 Neuss 21 (DE)
(74) Vertreter: Kühn, Hans-Christian

(56) Entgegenhaltungen:
- EP-A- 0 355 562
- DE-A- 2 420 351
- DATABASE WPIL Week 8128, Derwent Publications Ltd., London, GB; AN 81-50842D

## Beschreibung

Die Erfindung betrifft Zubereitungen zur Verwendung als Adhäsivkomponente bei der Restauring von Zahnhartsubstantz.

Ein besonders gravierendes Problem in der konservierenden Zahnheilkunde besteht in der dauerhaften, randspaltfreien Verbindung von Kunststoff-Füllungsmaterialien mit der Zahnhartsubstanz (Dentin und Zahnschmelz). Im Dentalbereich werden härtende, polymere Materialien als Füllungsmaterialien bei Zahnreparaturen verwendet. Als härtende, polymere Materialien werden im allgemeinen Füllungen auf Acrylat-Basis bevorzugt, die während der Aushärtung schrumpfen und so zur Randspaltbildung beitragen.

Diese polymeren Füllungen haben weiterhin den Nachteil, daß sie schlecht am Dentin haften bleiben. Um dieses Problem zu lösen, hat man bisher teilweise Unterschneidungen am Zahnbein (Dentin) vorgenommen; dazu war es erforderlich, über den angegriffenen Bereich hinaus beachtliche Mengen an frischem Dentin zu entfernen. Nach einer anderen Methode ätzt man das Dentin und die Schmelzoberfläche mit Säuren, wie z.B. Phosphorsäure, an und nimmt dann die Füllung vor.

Zur Verbesserung der Verbundhaftung zwischen Zahnhartsubstanz und Kunststoff-Füllungsmaterial wurden Adhäsiv-Zubereitungen beschrieben, deren Anbindung an die härtende Acrylat-Füllung durch Copolymerisation mit dem adhäsiv wirksamen Bestandteil der Zubereitung erreicht wird. Diese Adhäsive enthalten deshalb alle Acrylat-Gruppen. Vielfach wurde versucht, das gut zugängliche, preiswerte und gut verträgliche 2-Hydroxyethylmethacrylat (HEMA) als adhäsiv wirksamen Bestandteil einzusetzen. Es zeigte sich jedoch, daß HEMA sich allein nicht zu einem wirksamen Dentaladhäsiv formulieren ließ, sondern daß immer ein spezielles Additiv zugegeben werden mußte.

Munksgaard und Asmussen beschreiben wäßrige Mischungen aus HEMA und Glutardialdehyd als Additiv (J. Dent. Res. 63 (1984) 1087).

Mit abnehmender Menge an Glutardialdehyd nimmt die Klebewirkung deutlich ab und ist bei Weglassen des Aldehydes nicht mehr vorhanden. In der Literatur wird die mangelnde Zellverträglichkeit, z.B. auf humane Gingivafibroblasten dieses Produktes, welche durch den Aldehyd hervorgerufen wird, bemängelt (A. Brauner, W. Krüger, P. Kaden, F. Lampert, C. Mittermayer, Dtsch. Zahnärztl. Z. 43 (1988) 396).

Die handelsüblichen Adhäsiv-Zubereitungen der Firma Kuraray (Clearfil Photobond, Clearfil New Bond, Clearfil Binding Agent) enthalten neben HEMA Additive vom Typ Dihydrogenphosphat oder Hydrogenphosphat-Gruppen enthaltender Methacrylate. Diese Additive bedingen letztlich die adhäsive Wirkung (E. Asmussen, E.C. Munksgaard, in "Posterior Composite Resin Dental Restorative Materials", Peter Szulc Publishing Co. 1985, Seite 217). Die speziellen Methacrylate dieser Art sind nur in aufwendigen Synthesen darstellbar.

Es wurde nun eine Zubereitung zur Verwendung als Adhäsivkomponente für die Zahnhartsubstanz gefunden, enthaltend
A) 10 bis 90 Gew.-% Hydroxyalkyl(meth)acrylat der Formel

   H₂C=CR¹-CO-O-R²-OH (I),

   in der
   - R¹: für Wasserstoff oder Methyl und
   - R²: für einen zweiwertigen Alkylrest mit 2 bis 6 C-Atomen
   stehen,
B) 1 bis 50 Gew.-% N-Hydroxyalkylcarbonamid der Formel

   R³-CO-NR⁴-R⁵-OH (II),

   in der
   - R³: für Wasserstoff oder einen einwertigen Alkylrest mit 1 bis 3 C-Atomen,
   - R⁴: für Wasserstoff oder Methyl und
   - R⁵: für einen zweiwertigen Kohlenwasserstoffrest mit 2 bis 6 C-Atomen
   stehen,
   und gegebenenfalls
C) 0,01 bis 2,0 Gew.-% Initiatoren und/oder
   0,01 bis 4,0 Gew.-% Coaktivatoren
   und/oder
D) 5 bis 90 Gew.-% Lösungsmittel
   und/oder
E) 0,3 bis 80 Gew.-% (Meth)acrylsäureester, die Vernetzungen ausbilden können,
wobei die Summe aller Bestandteile 100 Gew.-% ergibt.

(Meth)acrylsäure-Derivate im Rahmen dieser Erfindung sind Derivate der Methacrylsäure oder Acrylsäure.

Zweiwertige Alkylrest R² sind gerad- oder verzweigtkettige aliphatische Alkylreste mit 2 bis 6 C-Atomen. Geeignete Hydroxyalkyl(meth)acrylate A sind demnach z.B. 4-Hydroxybutylmethacrylat, 4-Hydroxybutylacrylat und 3-Hydroxypropylmethacrylat. Bevorzugt seien genannt 2-Hydroxypropylmethacrylat und 2-Hydroxyethylacrylat. Besonders bevorzugt ist 2-Hydroxyethylmethacrylat.

Die erfindungsgemäßen Zubereitungen enthalten im allgemeinen 10 bis 90 Gew.-% Hydroxyalkyl(meth)acrylate, bevorzugt 15 bis 60 Gew.-% und besonders bevorzugt 20 bis 40 Gew.-%.

Bei den N-Hydroxyalkylcarbonamiden B gemäß Formel (II) stehen R³ für Wasserstoff, Methyl, Ethyl, Propyl oder Isopropyl, R⁴ für Wasserstoff oder Methyl und R⁵ für einen zweiwertigen aliphatischen, aromatischen, gerad- oder verzweigtkettigen Kohlenwasserstoffrest mit 2 bis 6 C-Atomen. Als geeignete N-Hydroxyalkylcarbonamide seien genannt
N-2-Hydroxyethylacetamid H₃C-CO-NH-CH₂-CH₂-OH ,
N-2-Hydroxypropylacetamid H₃C-CO-NH-CH₂-CHCH₃-OH ,
N-3-Hydroxypropylformamid H-CO-NH-CH₂-CH₂-CH₂-OH ,
N-2-Hydroxyethylbuttersäureamid H₇C₃-CO-NH-CH₂-CH₂-OH ,
N-2-Hydroxypropyl-N-methylacetamid H₃C-CO-NCH₃-CH₂-CHCH₃-OH
und
N-2-Hydroxyethyl-N-methylformamid H-CO-NCH₃-CH₂-CH₂-OH .

Bevorzugt sind
N-p-Hydroxyphenylformamid 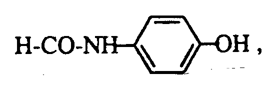
N-p-Hydroxyphenylacetamid 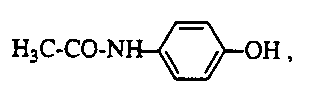
N-2-Hydroxyethylpropionamid H₅C₂-CO-NH-CH₂-CH₂-OH und
N-5-Hydroxypentylformamid H-CO-NH-(CH₂)₅-OH .

Besonders bevorzugte N-Hydroxyalkylcarbonamide B sind
N-5-Hydroxypentylacetamid H₃C-CO-NH-(CH₂)₅-OH ,
N-2-Hydroxypropylpropionamid H₅C₂-CO-NH-CH₂-CHCH₃-OH ,
N-2-Hydroxyethyl-N-methylpropionamid H₅C₂-CO-NCH₃-CH₂-CH₂-OH ,
N-3-Hydroxypropyl-N-methylacetamid H₃C-CO-NCH₃-CH₂-CH₂-CH₂-OH ,
N-3-Hydroxypropylacetamid H₃C-CO-NH-CH₂-CH₂-CH₂-OH ,
N-3-Hydroxypropylpropionamid H₅C₂-CO-NH-CH₂-CH₂-CH₂-OH
und
N-3-Hydroxypropylbuttersäureamid H₇C₃-CO-NH-CH₂-CH₂-CH₂-OH .

Im allgemeinen enthalten die erfindungsgemäßen Zubereitungen 1 bis 50 Gew.-% N-Hydroxyalkylcarbonamide, bevorzugt sind 5 bis 30, besonders bevorzugt 10 bis 20 Gew.-%.

N-Hydroxyalkylcarbonamide sind an sich bekannt Verbindungen und lassen sich z.B. durch Acylierung von Hydroxyalkylaminen mit Carbonsäureestern, insbesondere von Carbonsäuremethyl- oder Carbonsäureethylestern, in einem organischen Lösungsmittel wie z.B. Methanol oder Ethanol, oder auch lösungsmittelfrei zwischen 60 und 100°C herstellen. Hydroxyalkylformamide lassen sich grundsätzlich auch durch Carbonylierung der Aminoalkohole mit Kohlenmonoxid unter Druck erhalten (US 2 793 211). N-p-Hydroxyphenylformamid läßt sich außerdem aus p-Aminophenol und Ameisensäure in Wasser synthetisieren (Beilsteins Handbuch der Organischen Chemie, 4. Aufl., Band 13, 1930, S. 459).

Die Lösungsmittel im Rahmen der vorliegenden Erfindung sollen die Komponenten lösen und sollen, durch die Anwendung bedingt, nicht toxisch sein. Bevorzugt seien Wasser und flüchtige organische Lösungsmittel wie Methanol, Ethanol, Propanol, Isopropanol, Aceton, Methylethylketon, Essigsäuremethyl- oder -ethylester bzw. Tetrahydrofuran genannt.

Im allgemeinen setzt man 5 bis 90 Gew.-%, bevorzugt 20 bis 80 Gew.-% und besonders bevorzugt 40 bis 75 Gew.-%, des Lösungsmittels ein.

Initiatoren im Rahmen der vorliegenden Erfindung sind Radikalbildner, die eine radikalische Polymerisation auslösen. Bevorzugt sind Fotoinitiatoren, die unter der Einwirkung von Licht, beispielsweise UV-Licht, sichtbarem Licht oder Laserlicht, eine radikalische Polymerisation auslösen.

Die sogenannten Fotopolymerisationsinitiatoren sind an sich bekannt (Houben-Weyl, Methoden der organischen Chemie, Band E20, Seite 80 ff, Georg Thieme Verlag Stuttgart 1987). Vorzugsweise handelt es sich um Mono- oder Dicarbonylverbindungen, wie Benzoin und dessen Derivate, insbesondere Benzoinmethylether, Benzil und Benzilderivate, beispielsweise 4,4-Oxidibenzil und andere Dicarbonylverbindungen wie Diacetyl, 2,3-Pentandion und α-Diketo-Derivate des Norbornans und substituierter Norbornane, Metallcarbonyle wie Pentacarbonylmangan oder Chinone wie 9,10-Phenanthrenchinon und Naphthochinon. Besonders bevorzugt ist Campherchinon.

Die erfindungsgemäßen Zubereitungen enthalten im allgemeinen 0 bis 2 Gew.-Teile, bevorzugt 0,1 bis 0,5 Gew.-Teile des Initiators, bezogen auf 1 Gew.-Teil enthaltener polymerisierbarer (Meth)acrylate A und E.

Enthält eines der mit der erfindungsgemäßen Adhäsivkomponente im Kontakt stehenden Fügeteile bereits einen Initiator der beschriebenen Art, kann auf den Initiator in der Adhäsivkomponente auch ganz verzichtet werden.

Es kann vorteilhaft sein, den erfindungsgemäßen Zubereitungen Coaktivatoren zuzusetzen, die die Polymerisationsreaktion beschleunigen. Bekannte Beschleuniger sind beispielsweise Amine wie p-Toluidin, Dimethyl-p-toluidin, Trialkylamine wie Trihexylamin, Polyamine wie N,N,N',N'-Tetraalkylalkylendiamin, Barbitursäure und Dialkylbarbitursäure.

Die Coaktivatoren werden im allgemeinen in einer Menge von 0 bis 4 Gew.-%, bevorzugt 0,2 bis 1 Gew.-%, bezogen auf die Menge an polymerisierbaren Verbindungen eingesetzt.

Es kann auch zweckmäßig sein, den erfindungsgemäßen Zusammensetzungen (Meth)acrylsäureester zuzusetzen, die Vernetzungen ausbilden können. (Meth)acrylsäureester, die Vernetzungen ausbilden können, enthalten im allgemeinen 2 oder mehr polymerisierbare aktive Gruppen im Molekül. Bevorzugt seien Ester der (Meth)acrylsäure mit 2- bis 5-wertigen Alkoholen mit 2 bis 30 Kohlenstoffatomen genannt. Besonders bevorzugt werden Alkoxy(meth)acrylate und Urethangruppen enthaltende (Meth)acrylate.

Beispielsweise seien (Meth)acrylsäureester der Formel 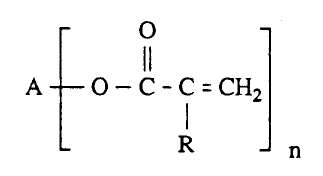
in der
- A: einen geradkettigen, verzweigten, cyclischen, aliphatischen, aromatischen oder gemischt aliphatisch-aromatischen Rest mit 2 bis 25 C-Atomen, der durch -O- oder NH-Brücken unterbrochen und mit Hydroxy, Oxy, Carboxy, Amino oder Halogen substituiert sein kann,
bedeutet,
- R: H oder Methyl bedeutet und
- n: für eine ganze Zahl von 2 bis 8, vorzugsweise 2 bis 4, steht,
genannt.

Vorzugsweise seien Verbindungen der folgenden Formeln genannt: 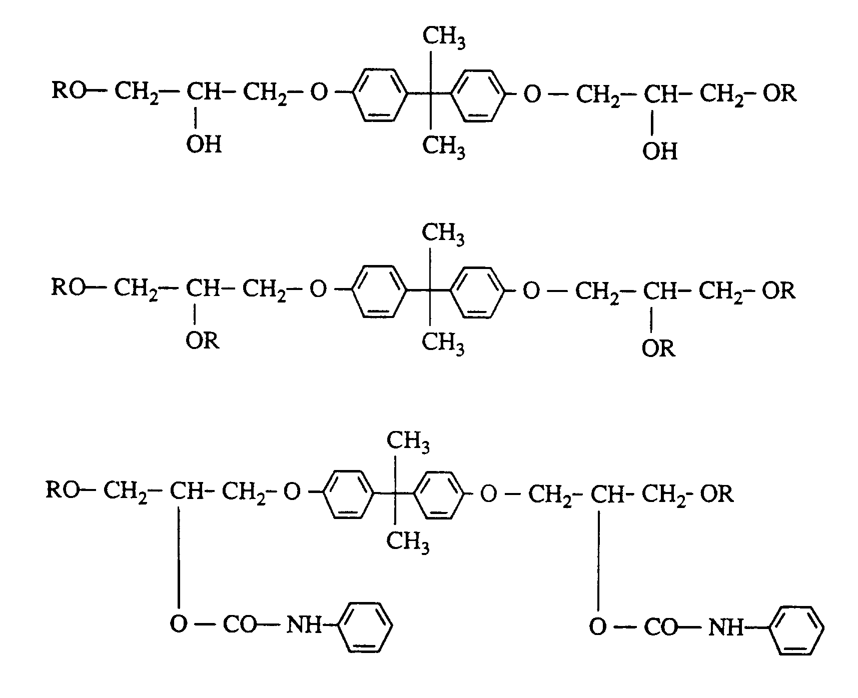 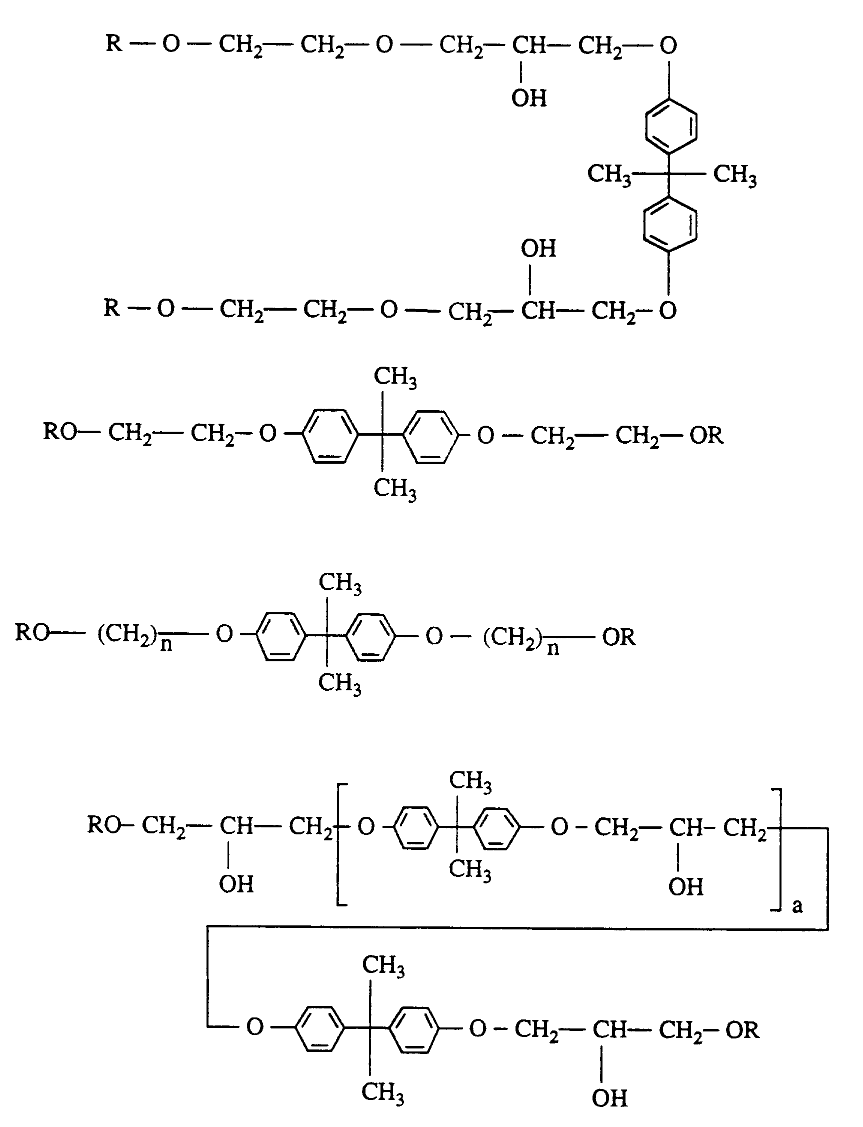
wobei a eine Zahl von 1 bis 4 bedeutet 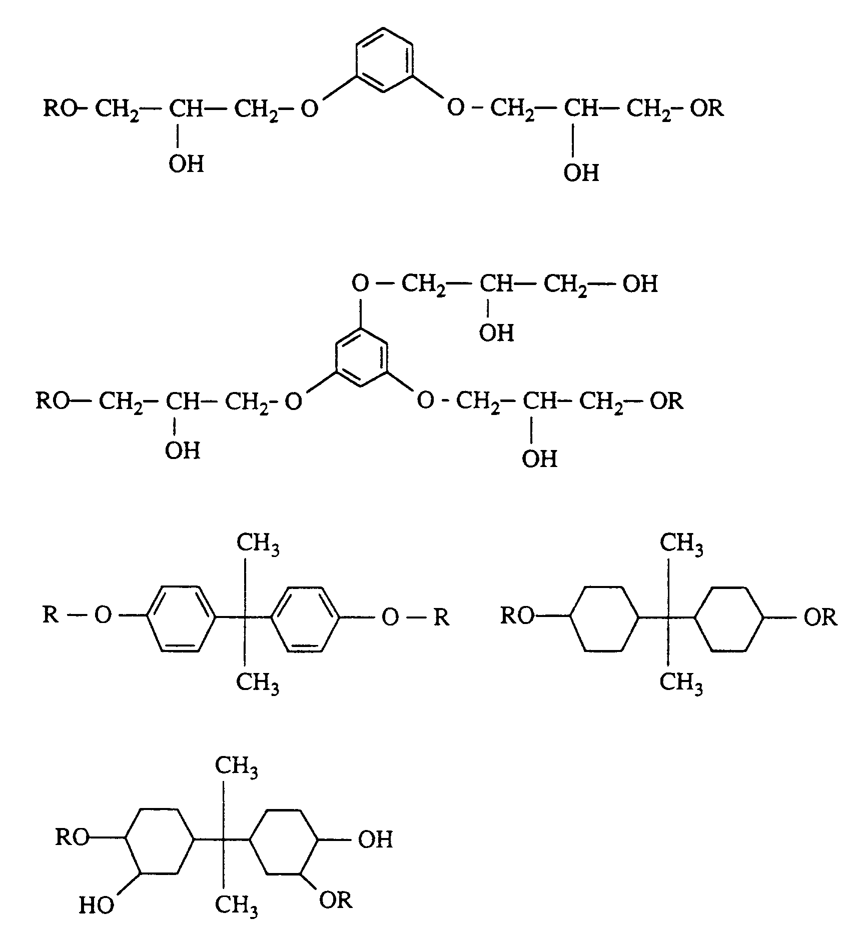 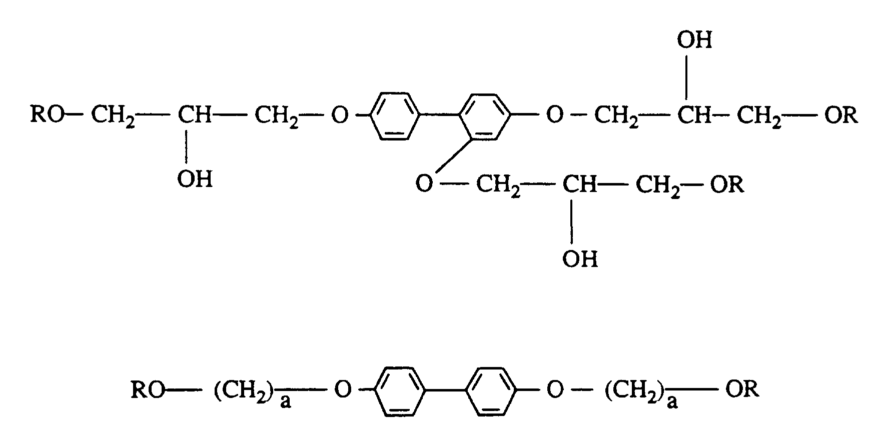
wobei a eine Zahl von 1 bis 4 bedeutet, 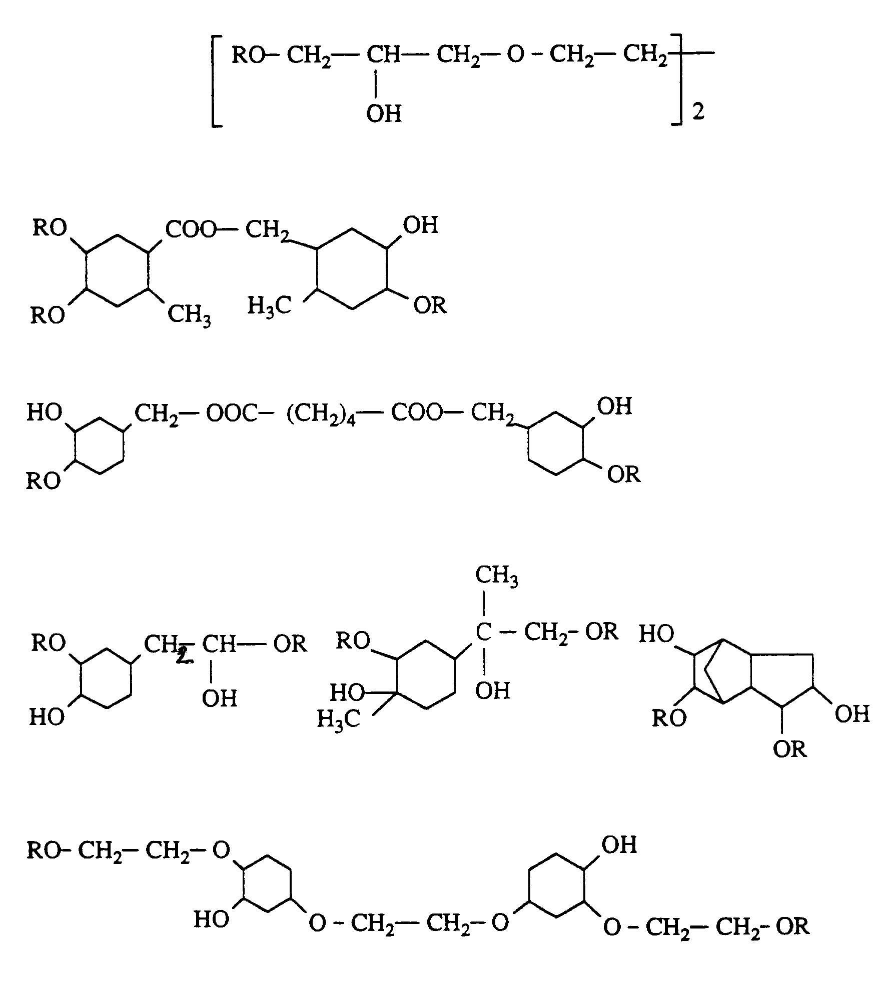 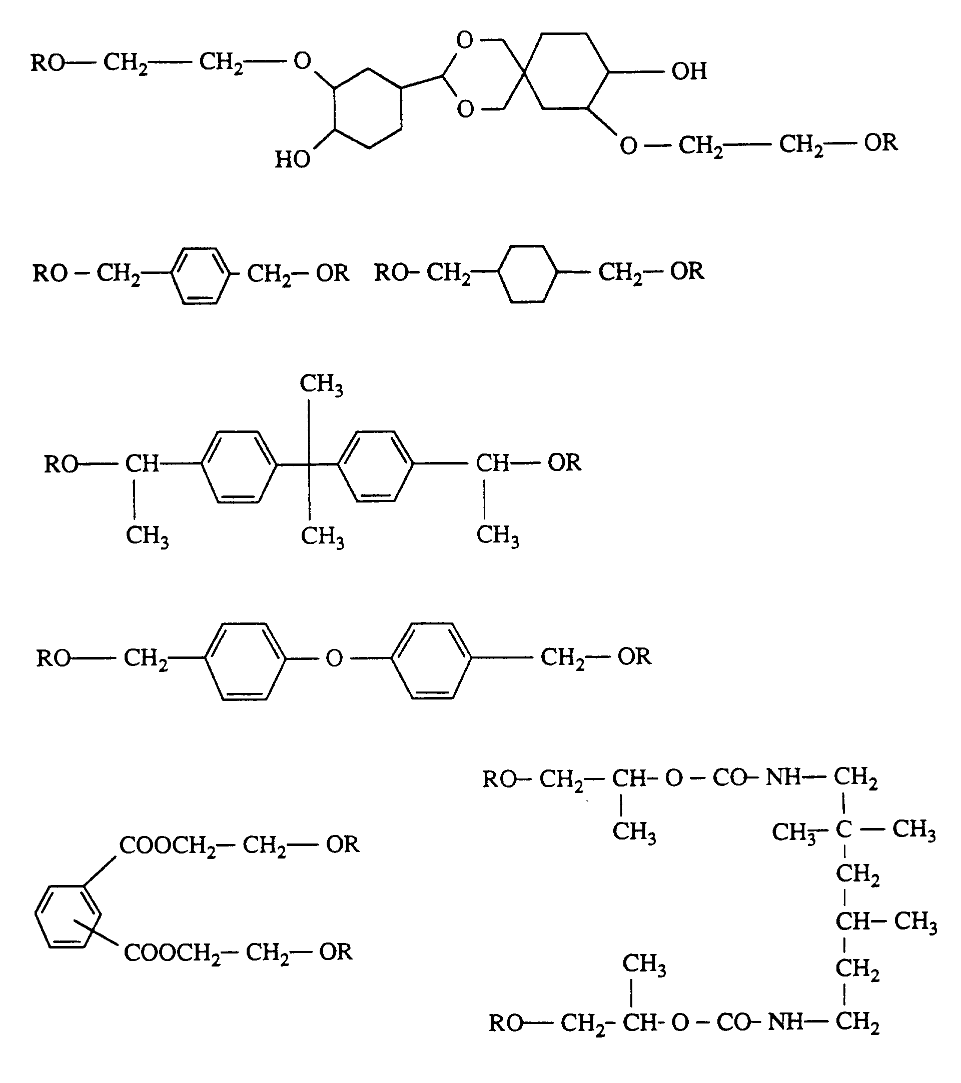
in der ortho-, meta- oder para-Form 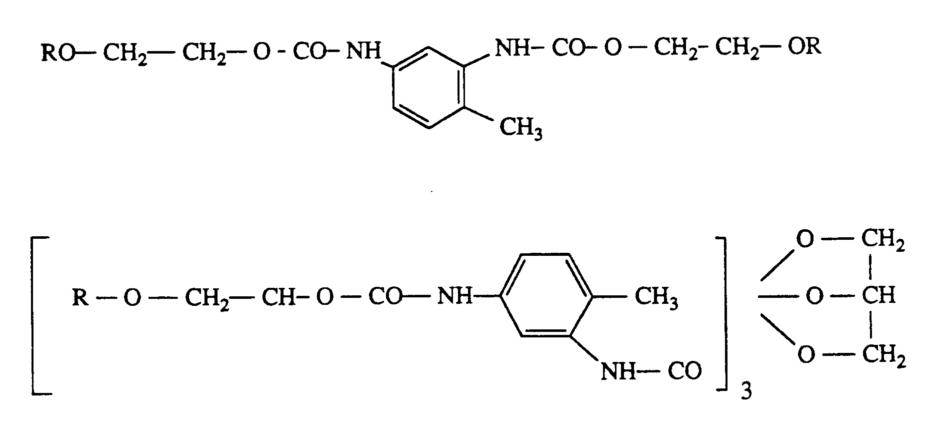
worin R für 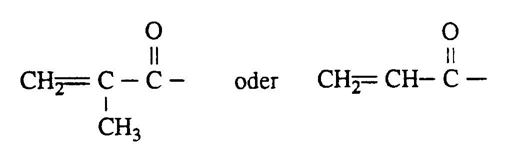
steht.

Außerdem seien Derivate des Tricyclodecans (EP-A 0 023 686) und Umsetzungsprodukte aus Polyolen, Diisocyanaten und Hydroxyalkylmethacrylaten (DE-A 37 03 120, DE-A 37 03 080 und DE-A 37 03 130) genannt. Beispielsweise seien die folgenden Monomeren genannt: 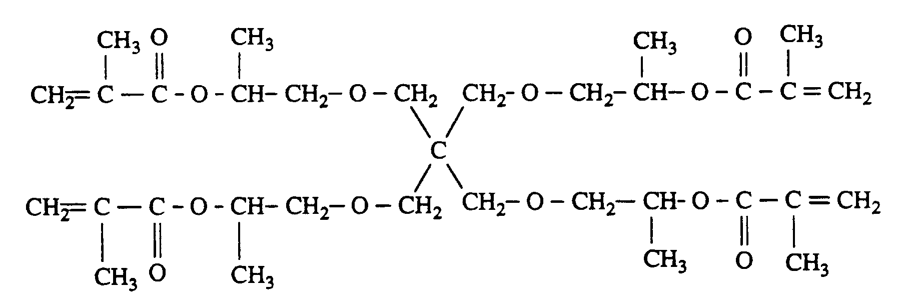 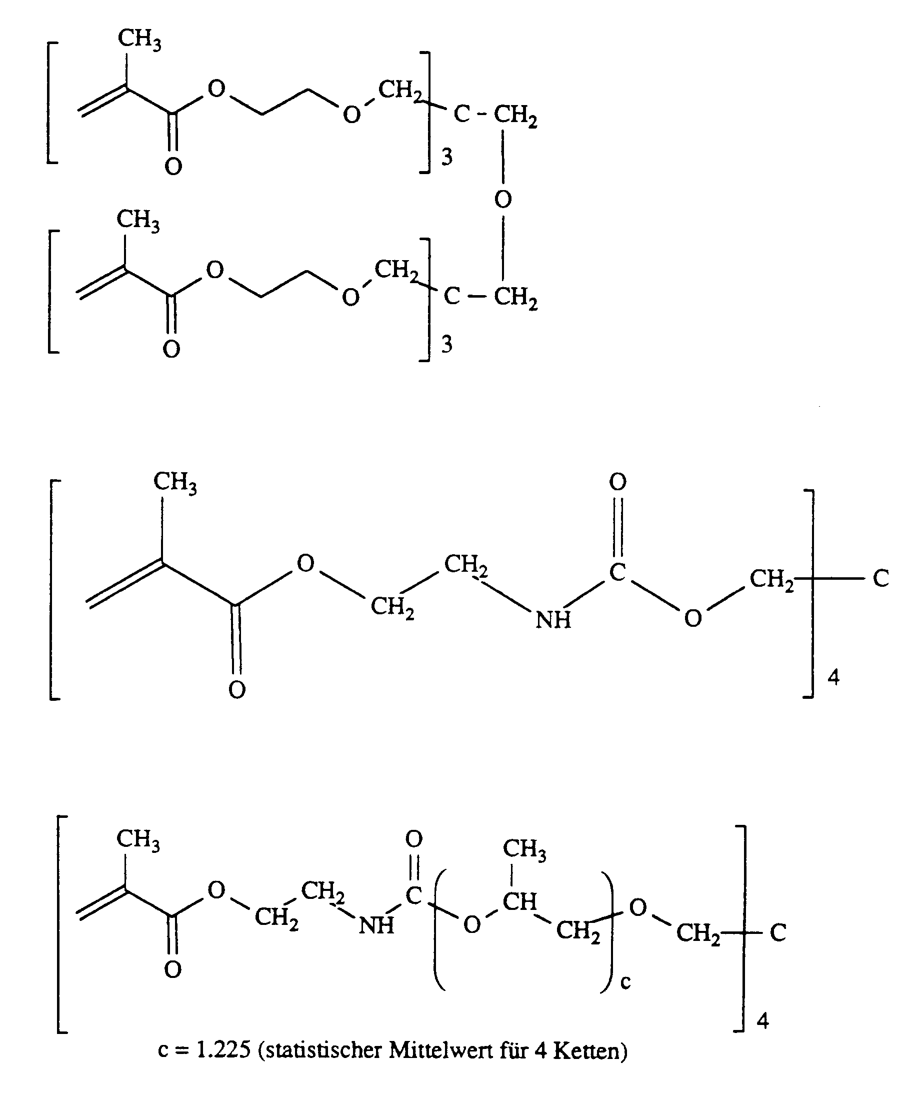 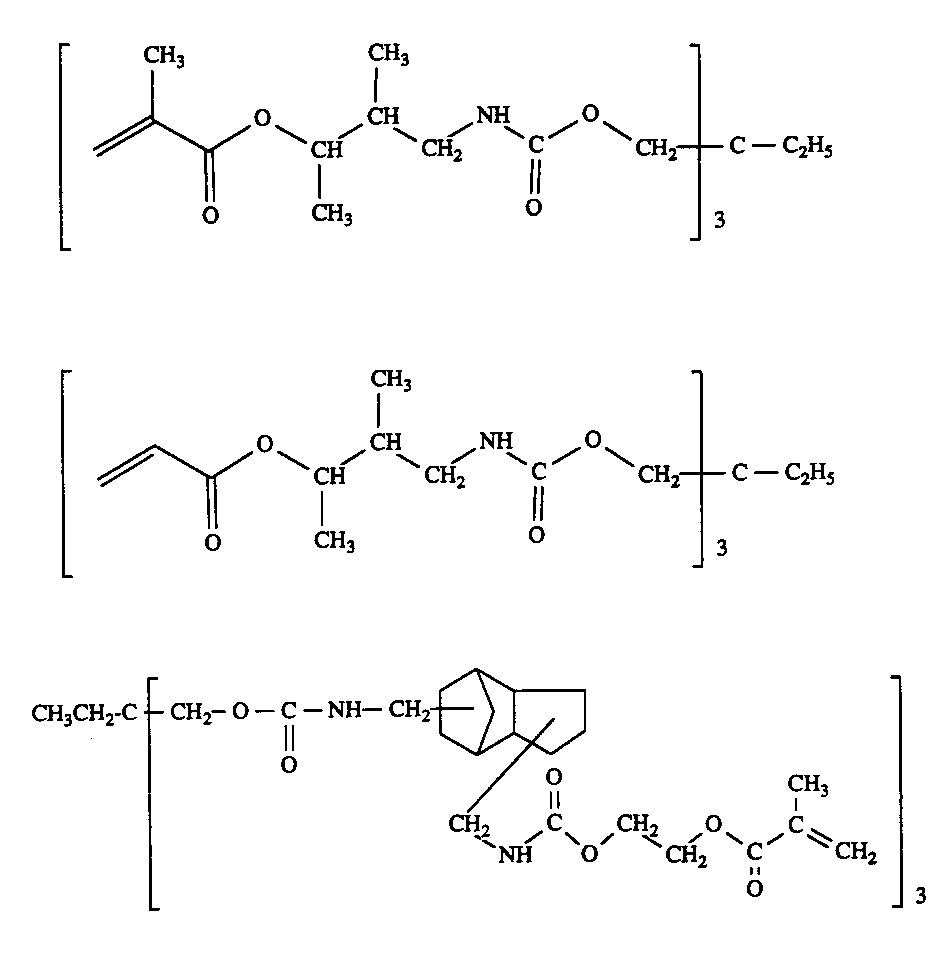 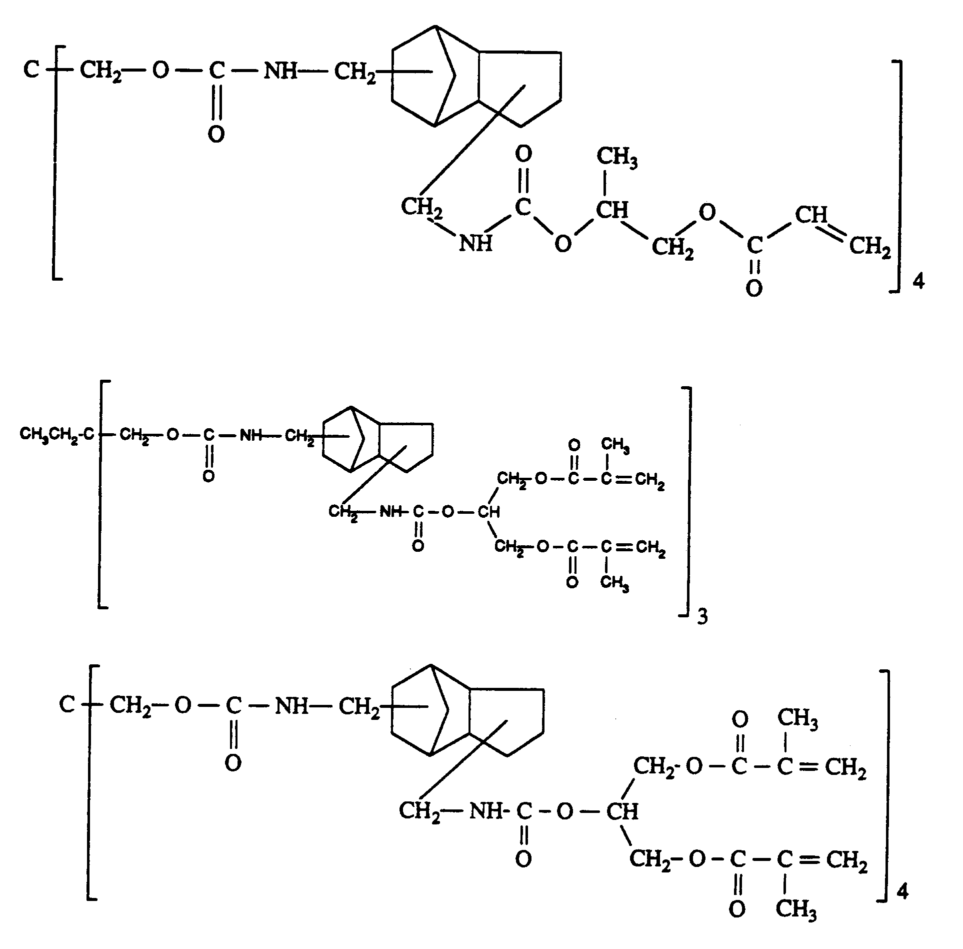 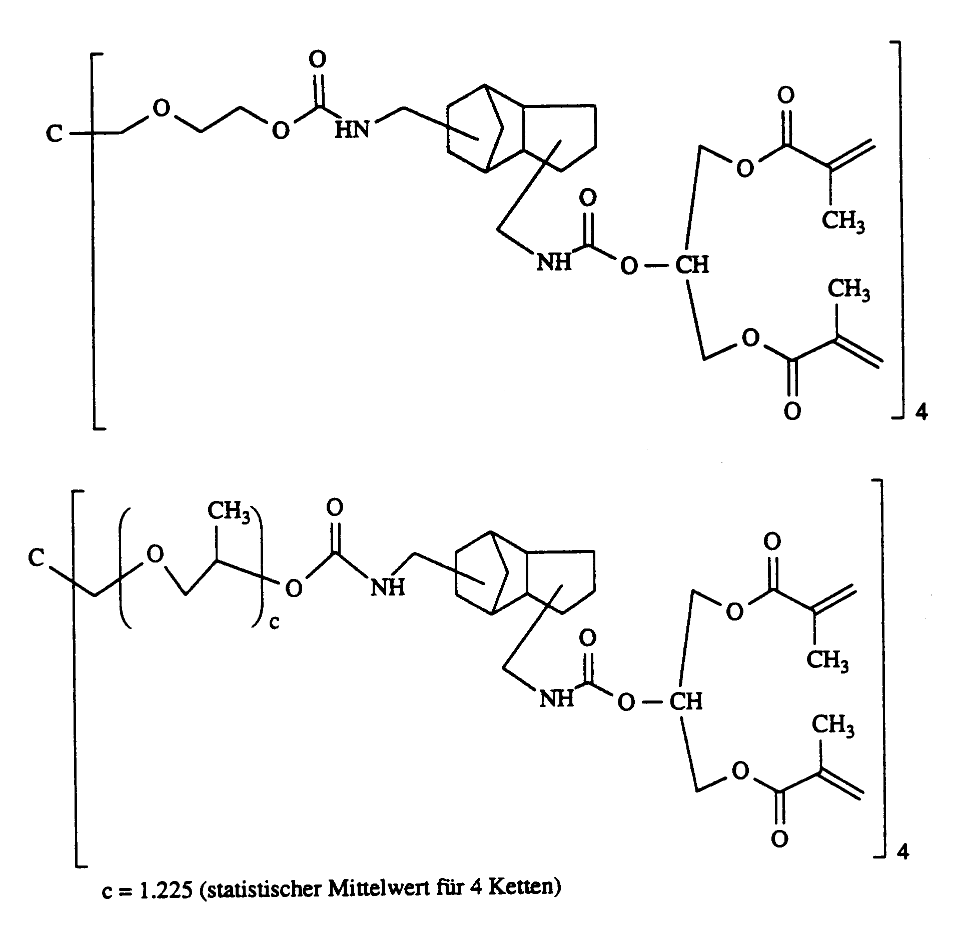 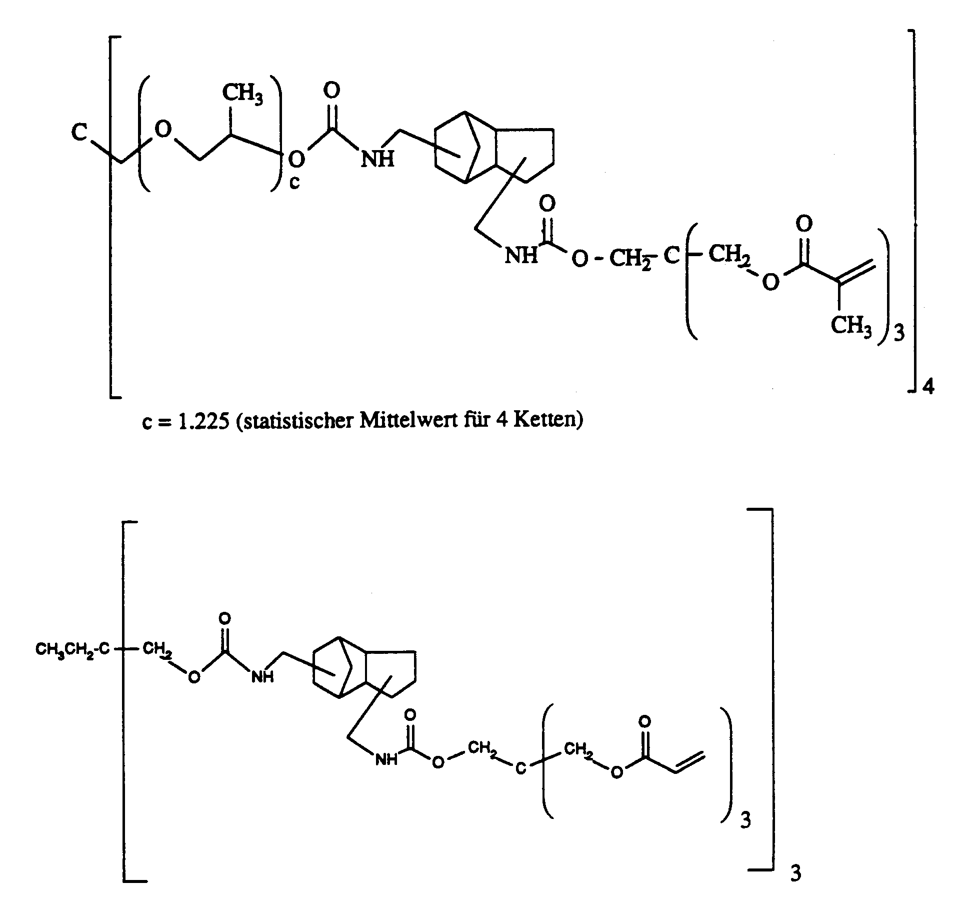 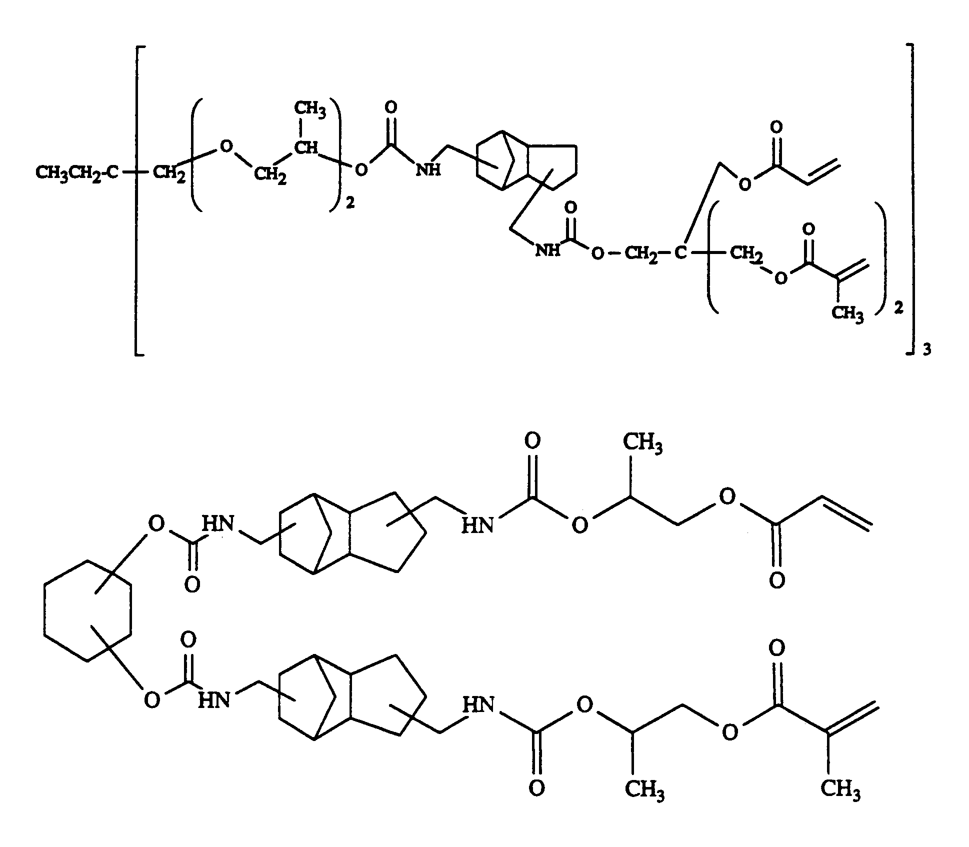 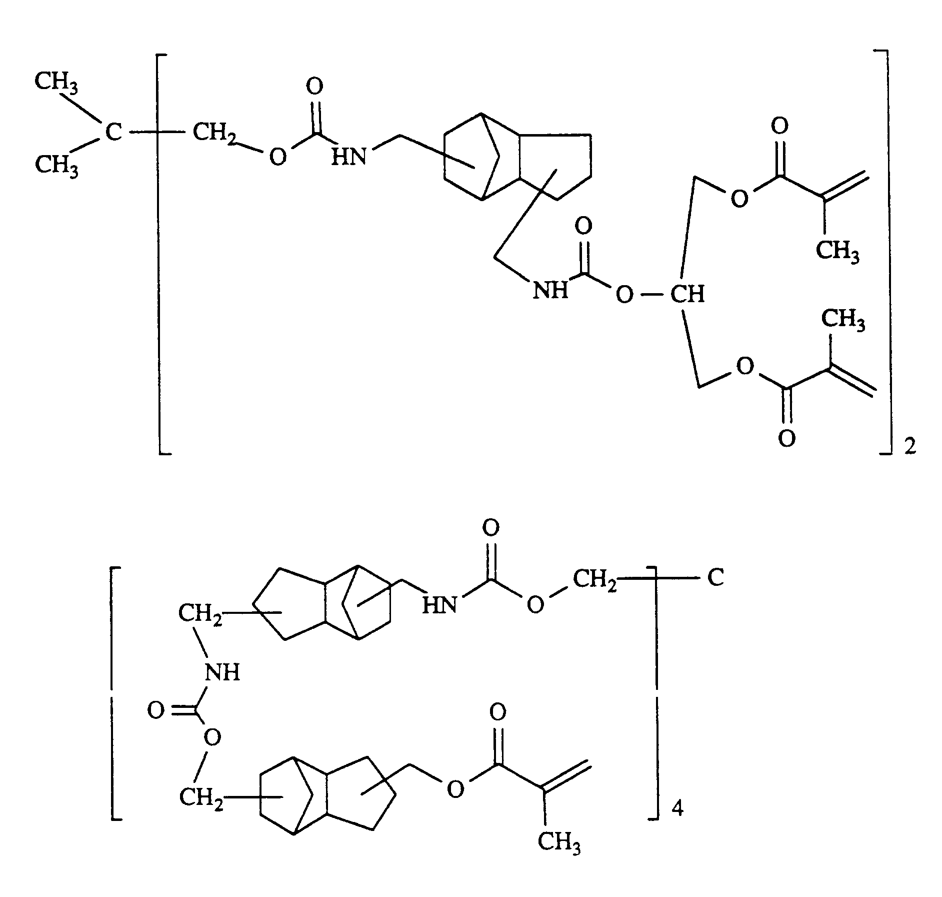 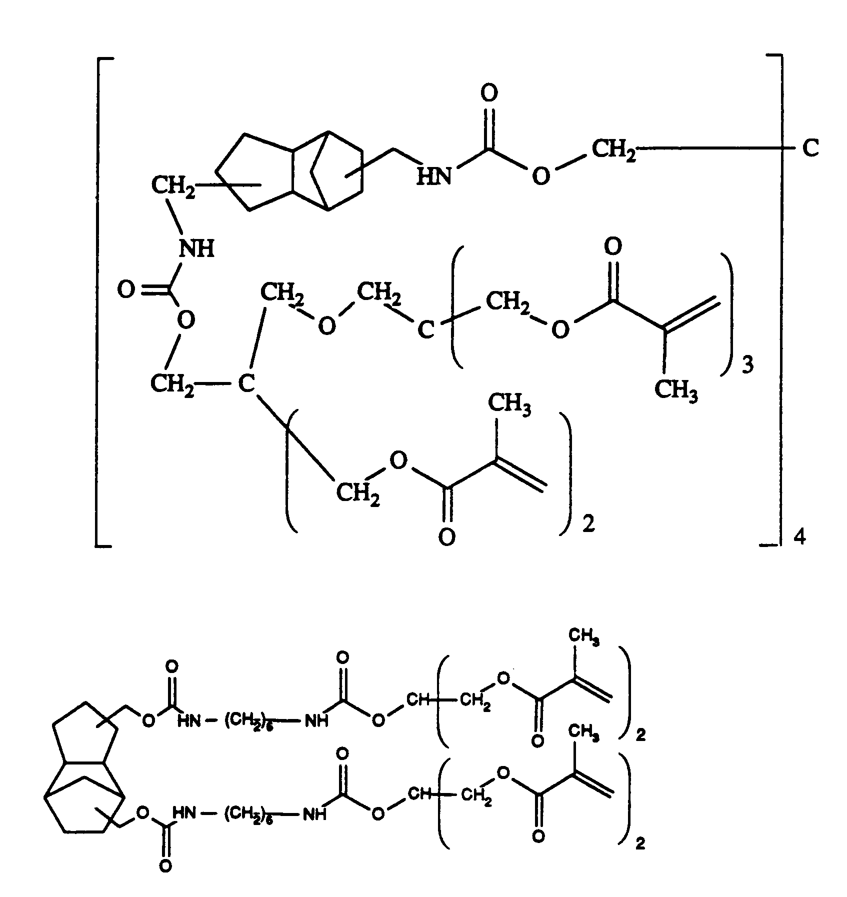 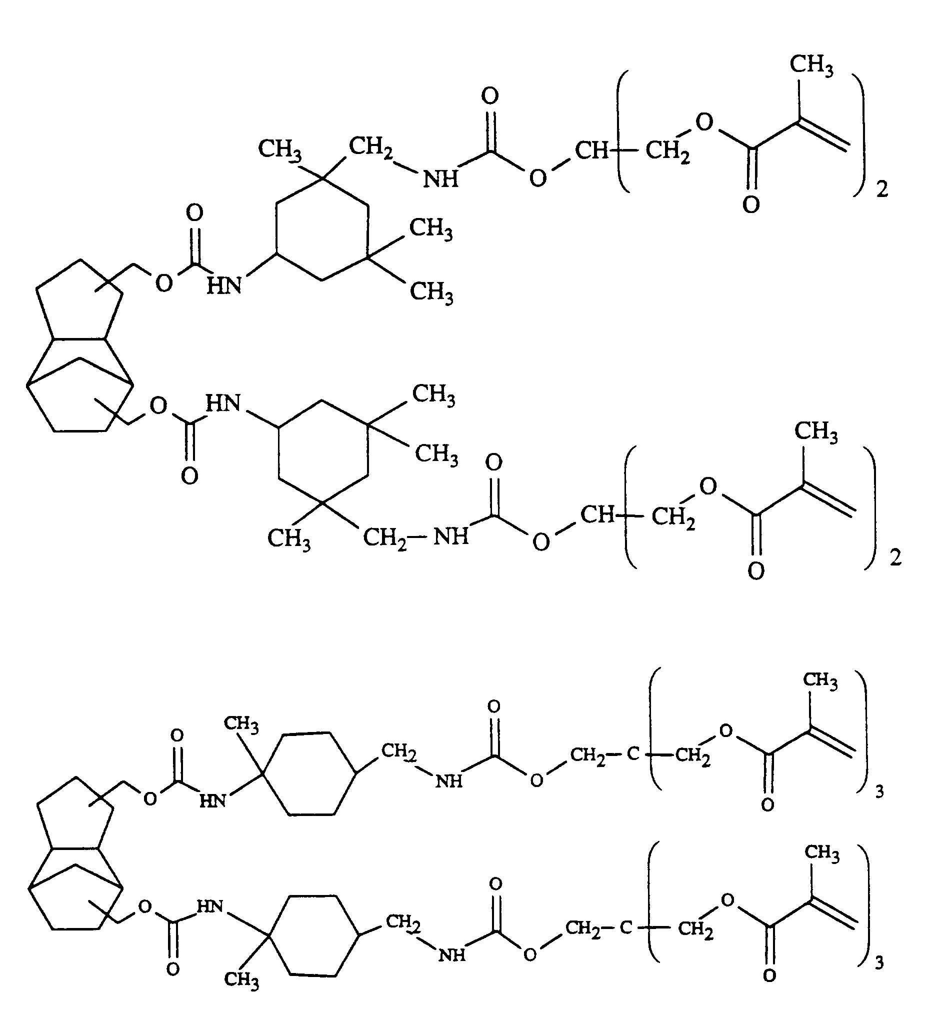 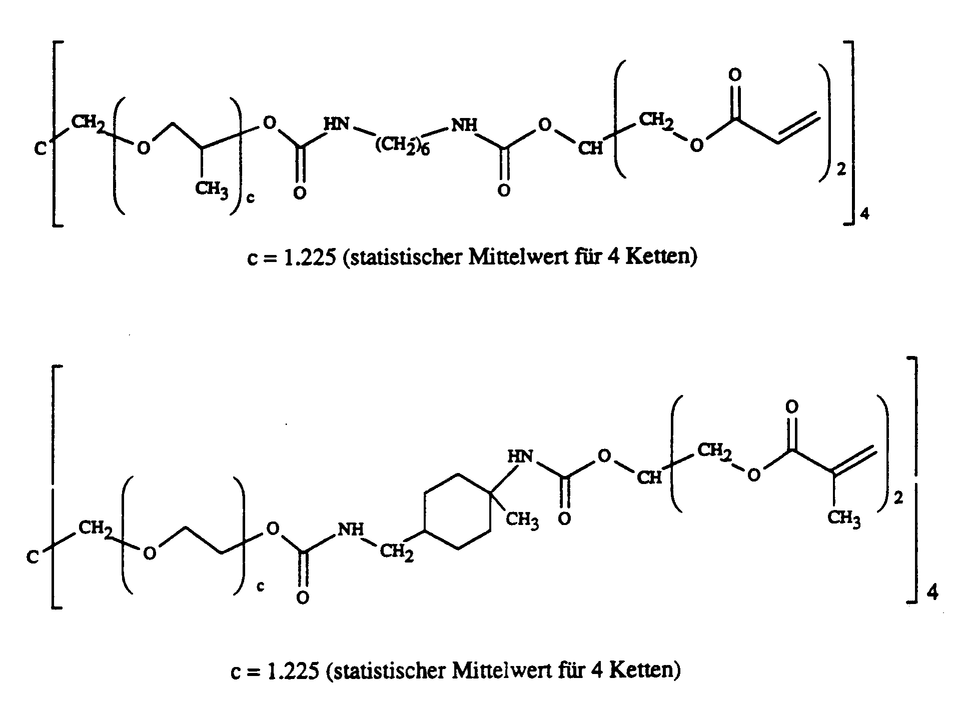 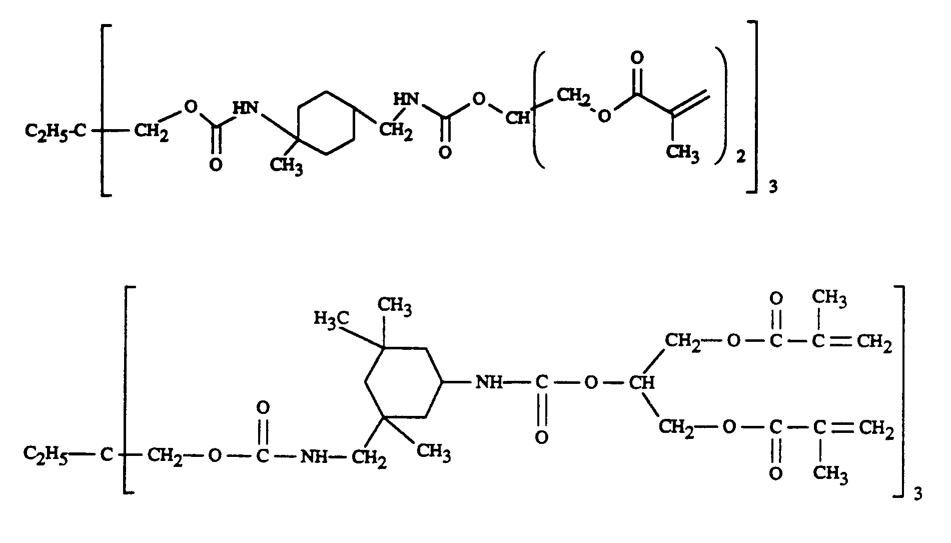
Als Methacrylsäureester bevorzugt wird das sogenannte Bis-GMA der Formel 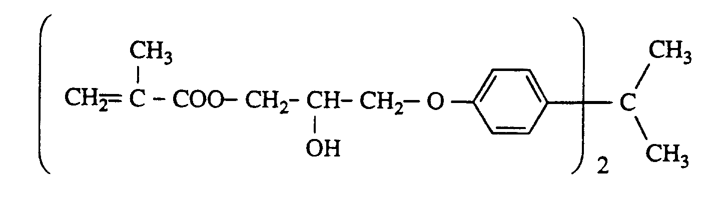
Selbstverständlich ist es möglich, Mischungen der verschiedenen (Meth)acrylsäureester, die Vernetzungen ausbilden können, einzusetzen. Beispielsweise seien Mischungen von 20 bis 70 Gew.Teilen Bis-GMA und 30 bis 80 Gew.-Teilen Triethylenglykoldimethacrylat genannt.

Die erfindungsgemäßen Zubereitungen enthalten im allgemeinen 0,3 bis 80 Gew.-Teile, bevorzugt 1 bis 50 Gew.-Teile und besonders bevorzugt 4 bis 30 Gew.-Teile der Methacrylsäureester, die Vernetzungen ausbilden können.

Als weitere Komponente können die erfindungsgemäßen Zusammensetzungen Füllstoffe enthalten. Als Füllstoffe werden feine Pulver bevorzugt, die einen Teilchendurchmesser im Bereich von 0,02 bis 100 »m (gegebenenfalls auch in einer polydispersen Verteilung) haben. Füllstoffe können im Dentalbereich übliche Füllstoffe (R.S. Baratz, J. Biomat. Applications, Vol 1, 1987, S. 316 ff) wie anorganische Gläser, Siliziumdioxid-, Aluminiumoxid- oder Quarzpulver sein.

Durch einen Anteil an Füllstoffen in den erfindungsgemäßen Zubereitungen entstehen Klebezemente, die besonders zur Befestigung von Brücken-, Kronen- und anderen Verblendmaterialien geeignet sind.

Der Anteil der Füllstoffe beträgt im allgemeinen 0 bis 80 Gew.-Teile, bevorzugt 15 bis 70 Gew.-Teile und besonders bevorzugt 20 bis 60 Gew.-Teile, bezogen auf die gesamte Zubereitung.

Die Adhäsivkomponenten gemäß dieser Erfindung können weiterhin bis zu 10 Gew.-% üblicher Zusätze wie Stabilisatoren, Inhibitoren, Lichtschutzmittel, Farbstoffe, Pigmente oder Fluoreszenzstoffe enthalten.

Die erfindungsgemäßen Zubereitungen können hergestellt werden, indem man die Komponenten A und B bzw. A bis E durch kräftiges Rühren mischt.

Die erfindungsgemäßen Zubereitungen können als Adhäsivkomponente zur Behandlung von Dentin und Zahnschmelz verwendet werden. Es muß als ausgesprochen überraschend bezeichnet werden, daß die erfindungsgemäßen Zubereitungen eine gute Verbundhaftung zwischen Zahnhartsubstanz und Füllungsmaterial ermöglichen. Asmussen und Munksgaard beschreiben, daß die Gegenwart eines reaktiven Aldehydes wie Glutaraldehyd notwendig ist, um mit HEMA ein Adhäsiv zu formulieren (J. Dent. Res. 63 (1984) 1087). Diese Autoren erklären die Wirkung des Aldehydes in seiner Reaktion mit der Proteinstruktur des Dentins und anschließender chemischer Anbindung von z.B. Hydroxyethylmethacrylat an das so modifizierte Dentin (US 4 593 054). Die Zubereitungen gemäß der vorliegenden Erfindung enthalten jedoch keine Komponenten, die mit der Zahnhartsubstanz unter den Bedingungen des Mundmilieus zu reagieren vermögen. Insbesondere sind die N-Hydroxyalkylcarbonamide unter diesen Bedingungen völlig unreaktiv gegenüber der Zahnhartsubstanz und dem eingesetzten Hydroxyalkyl(meth)acrylat. Auch enthalten die erfindungsgemäßen Zubereitungen keine weiteren Komponenten oder chemische Strukturbestandteile, die nach dem Stand der Technik in der Lage wären, Anbindungen zur Zahnhartsubstanz zu ermöglichen.

Die erfindungsgemäßen Zubereitungen ermöglichen eine feste und dauerhafte Verbindung von Zahnhartsubstanz und Füllungsmaterial auf Basis leicht zugänglicher, preisgünstiger und toxikologisch unbedenklicher Bestandteile.

In einer besonderen Ausführungsform konditioniert man Zahnschmelz und Dentin vor der Behandlung mit der erfindungsgemäßen Zubereitung mit einer Flüssigkeit mit einem pH-Wert im Bereich von 0,1 bis 3,5.

Diese enthält im allgemeinen Säuren mit einem pK_{S}-Wert Kleiner als 5 und gegebenenfalls eine amphotere Aminoverbindung mit einem pK_{S}-Wert im Bereich von 9,0 bis 10,6 und einem pK_{B}-Wert im Bereich von 11,5 bis 12,5. Folgende Säuren können z.B. in der Konditionierflüssigkeit enthalten sein:
Phosphorsäure, Salpetersäure, Brenztraubensäure, Zitronensäure, Oxalsäure, Ethylendiamintetraessigsäure, Essigsäure, Weinsäure, Äpfelsäure, Maleinsäure.

Als amphotere Aminoverbindungen seien vorzugsweise Verbindungen der Formel 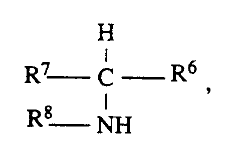
in der
- R⁶: für eine Carboxylgruppe steht,
- R⁷: Wasserstoff, gegebenenfalls durch Hydroxy, Thio, Methylthio, Carboxy, Amino, Phenyl, Hydroxyphenyl oder die Gruppen 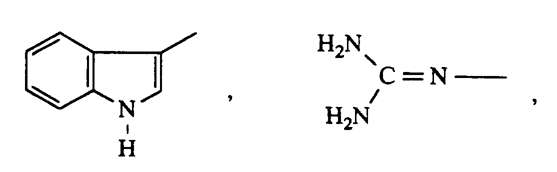 substituierter Niederalkylrest,
- R⁸: Wasserstoff oder Phenyl bedeutet und
wobei die Reste R⁶ und R⁸ durch einen Propylrest verbunden sein können, oder
in der
- R⁶: für Wasserstoff steht,
- R⁷: die Gruppe

-A-NH₃X ,

in der
- A: für einen zweibindigen Alkylenrest mit 1 bis 6 Kohlenstoffatomen und
- X: für Halogen steht, bedeutet und
- R⁸: Wasserstoff bedeutet,
genannt.

Beispielsweise seien die folgenden amphoteren Aminoverbindungen genannt: Glycin, Serin, Threonin, Cystein, Thysorin, Asparagin, Glutamin, Alanin, Valin, Leucin, Isoleucin, Prolin, Methionin, Phenylalanin, Tryptophan, Lysin, Arginin, Histidin, N-Phenylglycin, Ethylendiaminhydrochlorid, Ethylendiaminhydrobromid, Propylendiaminhydrochlorid, Propylendiaminhydrobromid, Butylendiaminhydrochlorid, Butylendiaminhydrobromid, Leucinhydrochlorid und Histidinhydrochlorid.

Weiterhin kann die Konditionierflüssigkeit Stoffe aus der Gruppe der Polyethylenglykole und Metallhydroxide enthalten. Insbesondere können die oben aufgeführten mehrbasigen Säuren auch als teilweise Metallsalze eingesetzt werden, solange freie Säurefunktionen verbleiben.

Konditionierflüssigkeiten, die mindestens eine der Säuren aus der Gruppe der Oxalsäure, Ethylendiamintetraessigsäure und Zitronensäure sowie gegebenenfalls eine amphotere Aminoverbindung aus der Gruppe des Glycins, N-Phenylglycins und Prolins enthalten, sind besonders bevorzugt.

Die Anwendung der erfindungsgemäßen Zubereitungen kann beispielsweise wie folgt durchgeführt werden:
Bei einer Zahnreparatur trägt man beispielsweise nach einer mechanischen Präparation des Zahnschmelzes und Dentins zuerst die Konditionierflüssigkeit auf, läßt eine kurze Zeit (beispielsweise 60 Sekunden) einwirken, spült das Zahnmaterial mit Wasser und trocknet es im Luftstrom. Dann trägt man die erfindungsgemäße Zubereitung beispielsweise mit einem kleinen Pinsel in einer dünnen Schicht auf und trocknet im Luftstrom. Nach der erfindungsgemäßen Behandlung trägt man die eigentliche Füllmasse, beispielsweise im Dentalbereich übliche Kunststoffüllungsmassen (K. Eichner, "Zahnärztliche Werkstoffe und ihre Verarbeitung", Bd. 2, S. 135 ff, Hüthig Verlag, 5. Aufl. 1985) auf.

In ähnlicher Weise können die erfindungsgemäßen Zubereitungen zur Befestigung von Kronen, Brücken und ähnlichen Hilfsmitteln verwendet werden.

### Beispiele:

Die erfindungsgemäßen Adhäsivkomponenten und die Vergleichskomponente werden durch intensives Vermischen der in folgenden Beispielen aufgeführten Bestandteile erzeugt.

### Beispiel 1

10,0g N-3-Hydroxypropylpropionamid
20,0g 2-Hydroxyethylmethacrylat
70,0g Wasser

### Beispiel 2

10,0g N-3-Hydroxypropylacetamid
20,0g 2-Hydroxyethylmethacrylat
70,0g Wasser

### Beispiel 3

10,0g N-p-Hydroxyphenylformamid
20,0g 2-Hydroxyethylmethacrylat
70,0g Wasser

### Beispiel 4

10,0g N-p-Hydroxyethyl-N-methylformamid
20,0g 2-Hydroxyethylmethacrylat
70,0g Wasser

### Beispiel 5

10,0g N-5-Hydroxypentylformamid
20,0 g 2-Hydroxyethylmethacrylat
70,0g Wasser

### Beispiel 6 (Vergleichsbeispiel)

22 g 2-Hydroxyethylmethacrylat
78 g Wasser

### Beispiel 7 (Anwendungstest, Bindungsfestigkeit)

Die Wirksamkeit und Eignung der Adhäsive (Beispiele 1 bis 5) wird überprüft durch Bestimmung der Scherbindungsfestigkeit auf Dentin und Schmelz. Es werden menschliche Zähne benutzt, die für max. drei Monate nach der Extraktion in 1 % Chloraminlösung aufbewahrt waren. Vor der Verwendung im Test werden die Zähne nach sorgfältiger Reinigung unter fließendem Wasser für mindestens drei und höchstens zehn Tage in physiologischer Kochsalzlösung gelagert. Am Tage vor der Verwendung im Bindungstest werden die Zähne einzeln mit Epoxidharz (®LEKUTHERM X20, Härter T3) in zylindrische Gummiformen von 25 mm Durchmesser und 12 mm Höhe eingebettet. Die Zähne werden durch Naßschleifen auf SiC-Papieren der Körnungen 240, 320, 400 und schließlich 600 soweit beschliffen, daß eine ausreichend große Schmelzoberfläche oder eine schmelznahe Dentinfläche zur Anbindung eines Kunststoffzylinders mit 3,5 mm Durchmesser freiliegt. Nach Abspülen mit entionisiertem Wasser und Trocknung im Luftstrom wird 30 Sekunden lang mit der Konditionierlösung und einem Wattepellet gereinigt, mit Wasser abgespült und getrocknet, bevor das Adhäsiv mit einem Pinsel aufgetragen, 30 Sekunden auf der Oberfläche belassen und dann im Druckluftstrom vorsichtig getrocknet wird. Die so vorbehandelte Probe wird in einer Einspannvorrichtung unter einer teilbaren Teflonform mit einer zylindrischen 3,5 mm weiten und 1 mm hohen Aufnahme festgeklemmt. Danach wird das Kunststoff-Füllungsmaterial ®Pekafill (U) mit einer Spritze in die zylindrische Form eingefüllt, mit einem O₂-undurchlässigen Strip abgedeckt und mit der Polymerisationsleuchte ®Translux CL (Kulzer) unter der aufliegenden Lichtaustrittsöffnung 60 Sekunden lang aktiviert Unmittelbar im Anschluß daran wird die Probe aus der Halterung entfernt. Die Teflonform wird abgenommen und die Probe wird für 15 Minuten in 23°C warmem Wasser gelagert bis zur Einleitung der Scherbelastung, die mit Hilfe eines Druckstempels parallel zu und dicht an der Oberfläche des eingebetteten Zahnes unter einer Vorschubgeschwindigkeit von 1 mm/Minute bis zur Abtrennung erfolgt. Die Scherbindungsfestigkeit, der Quotient aus Bruchkraft und Kontaktareal am Zahn, wird jeweils an 3 Proben bestimmt und als deren Mittelwert angegeben.

Die Ergebnisse sind in folgender Tabelle zusammengestellt:

| Zubereitung nach Beispiel Nr. | Scherbindungsfestigkeit auf Dentin [N/mm²] |
|---|---|
| 1 | 7,8 |
| 2 | 6,1 |
| 3 | 4,9 |
| 4 | 4,4 |
| 5 | 5,0 |
| 6 (Vergleich) | <2,0 |

Bei der lichtmikroskopischen Beurteilung der Frakturursache wurden hauptsächlich Kohäsivfrakturen im Dentin oder im Kunststoff beobachtet, d.h. die mit den erfindungsgemäßen Adhäsivkomponenten erzeugten Verbindungen waren fester als die verbundenen Fügeteile selbst. Dies zeigt die gute Leistungsfähigkeit der erfindungsgemäßen Adhäsivkomponenten.

## Patentansprüche

1. Zubereitung zur Verwendung als Adhäsivkomponente für die Zahnhartsubstanz, enthaltend
A) 10 bis 90 Gew.-% Hydroxyalkyl(meth)acrylat der Formel
H₂C=CR¹-CO-O-R²-OH (I),
in der
R¹ für Wasserstoff oder Methyl und
R² für einen zweiwertigen Alkylrest mit 2 bis 6 C-Atomen
stehen,
B) 1 bis 50 Gew.-% N-Hydroxyalkylcarbonamid der Formel
R³-CO-NR⁴-R⁵-OH (II),
in der
R³ für Wasserstoff oder einen einwertigen Alkylrest mit 1 bis 3 C-Atomen,
R⁴ für Wasserstoff oder Methyl und
R⁵ für einen zweiwertigen Kohlenwasserstoffrest mit 2 bis 6 C-Atomen
stehen,
und gegebenenfalls
C) 0,01 bis 2,0 Gew.-% Initiatoren und/oder
0,01 bis 4,0 Gew.-% Coaktivatoren
jeweils bezogen auf die Summe der Komponenten aus A und E,
und/oder
D) 5 bis 90 Gew.-% Lösungsmittel
und/oder
E) 0,3 bis 80 Gew.-% (Meth)acrylsäureester, die Vernetzungen ausbilden können,
wobei sich die Mengenangaben
zu den Komponenten, wenn nicht anders angegeben, auf die Gesamtzubereitung beziehen.

2. Zubereitung nach Anspruch 1, enthaltend
A) 15 bis 60 Gew.-% Hydroxyalkyl(meth)acrylat
B) 5 bis 30 Gew.-% N-Hydroxyalkylcarbonamid
C) 0,1 bis 5 Gew.-% Initiatoren und/oder
0,2 bis 1 Gew.-% Coaktivatoren,
jeweils bezogen auf die Summe der Komponenten aus A und E, und gegebenenfalls
D) 20 bis 80 Gew.-% Lösungsmittel
und/oder
E) 1 bis 50 Gew.-% (Meth)acrylsäureester, die Vernetzungen ausbilden können,
wobei sich die Mengenangaben
zu den Komponenten, wenn nicht anders angegeben, auf die Gesamtzubereitung beziehen.

3. Zubereitung nach Anspruch 1, enthaltend
A) 20 bis 40 Gew.-% Hydroxyalkyl(meth)acrylat
B) 10 bis 20 Gew.-% N-Hydroxyalkylcarbonamid
C) 0,1 bis 5 Gew.-% Initiatoren und/oder
0,2 bis 1 Gew.-% Coaktivatoren,
jeweils bezogen auf die Summe der Komponenten aus A und E, und gegebenenfalls
D) 40 bis 75 Gew.-% Lösungsmittel
und/oder
E) 4 bis 30 Gew.-% (Meth)acrylsäureester, die Vernetzungen ausbilden können,
wobei sich die Mengenangaben
zu den Komponenten, wenn nicht anders angegeben, auf die Gesamtzubereitung beziehen.

4. Zubereitung gemäß Anspruch 1, dadurch gekennzeichnet, daß als Komponente (B) N-3-Hydroxypropylpropionamid verwendet wird.

5. Zubereitung gemäß Anspruch 1, dadurch gekennzeichnet, daß als Komponente (B) N-3-Hydroxypropylacetamid verwendet wird.

6. Verwendung von Zubereitungen gemäß Anspruch 1 bei der Herstellung von Dentinadhäsiven.

## Claims

1. Formulation for use as an adhesive component for dental hard substance, comprising
A) 10 to 90 % by weight of hydroxyalkyl (meth)acrylate of the formula
H₂C=CR¹-CO-O-R²-OH (I)
in which
R¹ represents hydrogen or methyl and
R² represents a divalent alkyl radical having 2 to 6 C atoms,
B) 1 to 50 % by weight of N-hydroxyalkylcarboxamide of the formula
R³-CO-NR⁴-R⁵-OH (II)
in which
R³ represents hydrogen or a monovalent alkyl radical having 1 to 3 C atoms,
R⁴ represents hydrogen or methyl and
R⁵ represents a divalent hydrocarbon radical having 2 to 6 C atoms,
and if appropriate
C) 0.01 to 2.0 % by weight of initiators and/or
0.01 to 4.0 % by weight of coactivators
in each case based on the sum of the components from A and E,
and/or
D) 5 to 90 % by weight of solvent
and/or
E) 0.3 to 80 % by weight of (meth)acrylic acid esters which can form crosslinkings,
the amounts data for the components relating to the total formulation, unless stated otherwise.

2. Formulation according to Claim 1, comprising
A) 15 to 60 % by weight of hydroxyalkyl (meth)acrylate,
B) 5 to 30 % by weight of N-hydroxyalkylcarboxamide,
C) 0.1 to 5 % by weight of initiators and/or
0.2 to 1 % by weight of coactivators,
in each case based on the sum of the components from A and E,
and if appropriate
D) 20 to 80 % by weight of solvent
and/or
E) 1 to 50 % by weight of (meth)acrylic acid esters which can form crosslinkings,
the amounts data for the components relating to the total formulation, unless stated otherwise.

3. Formulation according to Claim 1, comprising
A) 20 to 40 % by weight of hydroxyalkyl (meth)acrylate,
B) 0 to 20 % by weight of N-hydroxyalkylcarboxamide,
C) 0.1 to 5 % by weight of initiators and/or
0.2 to 1 % by weight of coactivators,
in each case based on the sum of the components from A and E, and if appropriate
D) 40 to 75 % by weight of solvents
and/or
E) 4 to 30 % by weight of (meth)acrylic acid esters which can form crosslinkings,
the amounts data for the components relating to the total formulation, unless stated otherwise.

4. Formulation according to Claim 1, characterised in that N-3-hydroxypropylpropionamide is used as component (B).

5. Formulation according to Claim 1, characterised in that N-3-hydroxypropylacetamide is used as component (B).

6. Use of formulations according to Claim 1 in the preparation of dentine adhesives.

## Revendications

1. Préparation destinée à être utilisée comme adhésif pour la substance dentaire dure, contenant
A) 10 à 90 % en poids de (méth)acrylate d'hydroxyalkyle de formule
H₂C=CR¹-CO-O-R²-OH (I)
dans laquelle
R¹ représente de l'hydrogène ou un groupe méthyle et
R² est un reste alkyle divalent ayant 2 à 6 atomes de carbone,
B) 1 à 50 % en poids de N-hydroxyalkylcarboxamide de formule
R³-CO-NR⁴-R⁵-OH (II)
dans laquelle
R³ représente de l'hydrogène ou un reste alkyle monovalent ayant 1 à 3 atomes de carbone,
R⁴ est de l'hydrogène ou un groupe méthyle et
R⁵ est un reste hydrocarboné divalent ayant 2 à 6 atomes de carbone,
et le cas échéant
C) 0,01 à 2,0 % en poids d'initiateurs et/ou 0,01 à 4,0 % en poids de coactivateurs, dans chaque cas par rapport à la soie des composants A et E, et/ou
D) 5 à 90 % en poids de solvant et/ou
E) 0,3 à 80 % en poids d'esters d'acide (méth)acrylique, qui peuvent créer des réticulations,
les indications de quantités relatives aux composants, sauf spécification contraire, étant rapportées à la préparation totale.

2. Préparation suivant la revendication 1, contenant
A) 15 à 60 % en poids de (méth)acrylate d'hydroxyalkyle
B) 5 à 30 % en poids de N-hydroxyalkylcarboxamide
C) 0,1 à 5 % en poids d'initiateurs et/ou
0,2 à 1 % en poids de coactivateurs,
dans chaque cas par rapport à la somme des composés de A et E, et le cas échéant
D) 20 à 80 % en poids de solvant et/ou
E) 1 à 50 % en poids d'esters d'acide (méth)acrylique qui créent des réticulations,
les indications de quantités relatives aux composants, sauf spécification contraire, se rapportant à la préparation totale.

3. Préparation suivant la revendication 1, contenant
A) 20 à 40 % en poids de (méth)acrylate d'hydroxyalkyle
B) 10 à 20 % en poids de N-hydroxyalkylcarboxamide
C) 0,1 à 5 % en poids d'initiateurs et/ou
0,2 à 1 % en poids de coactivateurs,
dans chaque cas par rapport à la somme des composants de A et E,
et le cas échéant
D) 40 à 75 % en poids de solvant et/ou
E) 4 à 30 % en poids d'esters d'acide (méth)acrylique qui peuvent créer des réticulations,
les indications de quantités relatives aux composants, sauf spécification contraire, se rapportant à la préparation totale.

4. Préparation suivant la revendication 1, caractérisée en ce qu'on utilise comme composant (B) le N-3-hydroxypropylpropionamide.

5. Préparation suivant la revendication 1, caractérisée en ce qu'on utilise comme composant (B) le N-3-hydroxypropylacétamide.

6. Utilisation de préparations suivant la revendication 1 dans la production d'adhésifs pour dentine.
